# EUROPEAN PATENT APPLICATION

(11) **EP 3 995 088 A1**
(43) Date of publication of application: **11.05.2022**
(21) Application number: 19936400.1
(22) Date of filing: 19.12.2019
(51) Int. Cl.: A61B 17/122, A61B 17/128, A61B 17/00

(54) **CLAMP DEVICE USED IN COOPERATION WITH ENDOSCOPE, AND CLAMPING PORTION OF CLAMP DEVICE**

(30) Priority: 03.07.2019 CN 201910595081
(71) Applicant: Anrei Medical (Hangzhou) Co., Ltd., Hangzhou, Zhejiang 310018 (CN)
(72) Inventor: HU, Xiaogang, Hangzhou, Zhejiang 310018 (CN)
(74) Representative: Cabinet Chaillot
(86) International application number: PCT/CN2019/126560
(87) International publication number: WO 2021/000533

(57) **Abstract**

Disclosed is a clamp device used in cooperation with an endoscope. A clamping portion of the clamp device comprises clamping pieces (1), a clamp tube (2), a clamping piece fixing seat (3) and a connecting tube (4), wherein the clamping piece fixing seat (3) is arranged in the clamp tube (2) and can move forwards and backwards; the connecting tube (4) is fixed at the rear end of the clamping piece fixing seat (3); the tails of the two clamping pieces (1) are accommodated in the clamp tube (2), and the tails of the clamping pieces (1) are movably connected to the front end of the clamping piece fixing seat (3) by means of a deformation hole (141); stopping steps (220) are arranged on an inner wall of the tail of the clamp tube (2); when the two clamping pieces (1) perform clamping, protruding portions (142) at the tails of the clamping pieces (1) are connected to the stopping steps (220) in a clamping manner; the front end of the clamping piece fixing seat (3) extends forwards to form a connecting portion (300); the front portion of the connecting portion (300) respectively protrudes towards the left and right sides to form radial pin portions (310); and the tails of the two clamping pieces (1) are respectively arranged on the two radial pin portions (310) in a sheathed manner via the deformation hole (141), so as to achieve movable connection between the clamping pieces (1) and the clamping piece fixing seat (3). The clamp device used in cooperation with an endoscope has a reliable structure, is convenient to release, has fewer parts, is easier to manufacture, and is lower in cost.

## Description

### Technical Field

The disclosure relates to a clamp device in cooperation with an endoscope and a clamping portion thereof, to be specific, the disclosure is configured for suturing of a surgical wound under the endoscope to stop bleeding or prevent delayed bleeding, for temporarily clamping of tissues and for fixing other instruments, or for location marking and other purposes.

### Description of Related Art

With the development of endoscopic technology and other related technologies, endoscopic hemostasis has become the preferred treatment method for the treatment of gastrointestinal bleeding. At present, the commonly used endoscopic hemostasis methods include laser coagulation, electrocoagulation, local injection of drugs to hemostasis, drug spraying, and hemostatic clip clamping. Among these methods, the hemostatic clip clipping method has become the most effective and most clinically applicable method for non-surgical treatment of gastrointestinal bleeding due to its characteristics such as less trauma, fast hemostasis, low incidence of rebleeding, fewer complications, and definite curative effect. However, at present, the opening width of a hemostatic clip and the clamped length are maintained at 1:1, that is, a hemostatic clip with an opening of 12 mm will retain a length of 12 mm or greater after the final clamping. Increased retention length increases the risk of damaging the mucosa of the digestive tract. Further, a large number of parts are required, so that the structure and process become complex, and reliability thereby drops.

In the related art, the following clamping devices or hemostatic forceps are used to clamp tissues to close a wound, stop bleeding, or prevent delayed bleeding:

In the patent literature published in China with the patent application number CN201310272522.2, a release method through the fracture of a connecting piece is disclosed. The hemostatic clip fixes the clamping piece fixing seat and the clamping piece through the elastic reed on the tube wall of the clamp tube. In this fixing method, during instrument releasing, the clamping piece is exposed, so there is a risk of detaching, and the releasing process of the clamping piece is troublesome.

In the patent literature published in China with the patent application number CN201220250652.7, an elastic claw threaded into a separating ring with a flap ring is disclosed, and the separating ring is straightened when being released to achieve releasing. The flange of the elastic claw is engaged with the hemostatic clip fixed by the concave ring of the receiving tube. The force of the instrument to open the separating ring and the force applied to the separating ring are perpendicular to each other, so it takes more effort to detach the separating ring and the clamping piece. In addition, the receiving tube and the handle are released by squeezing and opening the claw, so such a structure is complicated and the operation is laborious.

The patent literature published in China with the patent application number CN201410222753.7 discloses a hemostatic clip in which a sliding groove is provided in the middle of a clamping arm, the opening and closing of the clip body are performed by reciprocating movement along the sliding groove, and the bending structure at the end of the sliding groove is locked. The clamping arm of the instrument is slotted, which increases the difficulty and costs of processing. Further, the special shape of the clamping arm makes the clamping arm less elastic so unstable clamping is provided. Moreover, in this instrument, the wire buckle is released from the clamping arm through the deformation of the wire buckle, but since the wire buckle has openings and gaps, the clip body may be accidentally detached when the clip body is repeatedly pushed, which affects the stability of use.

The patent literature published in US with the patent application number US8062311B2 discloses a hemostatic clip that requires a yoke to restrain the clip from advancing and stopping, and the yoke remains in the patient's body, and the clip tube has a barbed hole. The problem of inconvenience of use is still found in this instrument.

It can be seen from the above that at present, the releasing of a clamp device is implemented by breaking of the connecting member, the connecting piece, or other structural parts to achieve the separation of the clamping portion and the releasing portion, but broken residues may be found or fragment splashing may occur. Further, a large number of parts are required, the structure is complicated, and various functions are separately provided by different parts, as such, process reliability is poor, more parts remain in a patient's body, and the total length after clamping is relatively long. Therefore, the patent literature with the patent application number CN108013914A discloses an improved hemostatic clip used under an endoscope. The clamping portion thereof includes clamping pieces, a clamp tube, a stopping pin, a connecting pin, a clamping piece fixing seat, and a connecting tube configured for connecting a mandrel. The clamping piece fixing seat is arranged in the clamp tube and can move forwards and backwards, the connecting tube is fixed at a rear end of the clamping piece fixing seat, and the clamping pieces are elastic metal pieces. The tails of the clamping pieces are provided with a deformation hole with an opening, and the connecting pin penetrates through the deformation hole of the two clamping pieces and is then fixed to the front end of the clamping piece fixing seat. One side of the deformation hole at the tail of the clamping piece is provided with a protruding portion that is turned outwards. The tail of the inner wall of the clamp tube is provided with a circle of stopping steps. When the two clamping pieces perform clamping, the protruding portions of the tails of the clamping pieces are connected to the stopping steps in a clamping manner, and the stopping pin is fixed on the front of the clamp tube to restrict the clamping piece fixing seat from sliding out of the clamp tube. In this structure, reinforcing ribs are provided on the clamping pieces, the reinforcing ribs provide a large clamping force and may release no broken fragments. Therefore, a larger opening may be achieved while the length of the retained section is reduced. However, in such a structure, a large number of parts are required, the assembly structure is complicated, and the process steps are numerous. Further, the movable positioning between the clamping pieces and the clamping piece fixing seat is not reliable enough to facilitate a convenient and reliable surgical operation, and further improvement is still needed to lower the manufacturing costs.

### SUMMARY

### Technical Problems

In order to solve the above-mentioned technical problems, the purpose of the disclosure is to provide a clamp device and a clamping portion of the clamp device in cooperation with an endoscope which exhibit a reliable structure, may be conveniently released, require fewer parts, may be easily manufactured, and require reduced costs.

### Solutions to Problems

### Technical Solutions

To accomplish the foregoing purpose, the following technical solutions are adopted by the disclosure.

The disclosure provides a clamping portion of a clamp device in cooperation with an endoscope including clamping pieces, a clamp tube, a clamping piece fixing seat, and a connecting tube configured to be connected to a mandrel. The clamping piece fixing seat is arranged in the clamp tube and can move forwards and backwards, the connecting tube is fixed at a rear end of the clamping piece fixing seat, and the clamping pieces are elastic metal pieces. Tails of the two clamping pieces are accommodated in the clamp tube, and a deformation hole provided with an opening and outwardly-protruding protruding portions are arranged at the tails of the clamping pieces. The tails of the clamping pieces are movably connected to a front end of the clamping piece fixing seat by means of the deformation hole, and stopping steps are arranged on an inner wall of a tail of the clamp tube. When the two clamping pieces perform clamping, the protruding portions at the tails of the clamping pieces are connected to the stopping steps in a clamping manner. A connecting portion is arranged at the front end of the clamping piece fixing seat, and a front portion of the connecting portion protrudes towards left and right sides to form radial pin portions. The tails of the two clamping pieces are arranged on the two radial pin portions in a sheathed manner via the deformation hole, so as to achieve movable connection between the clamping pieces and the clamping piece fixing seat.

For the convenience of description, in the disclosure, the direction in which two clamping pieces in an axial direction of the clamp tube extend and expand is regarded as the front and the opposite direction is regarded as the back, and similarly, ends of other components are regarded as front ends or distal ends as close to a clamping portion, and vice versa as rear ends or proximal ends.

Preferably, two protection wing portions are arranged on the clamping piece fixing seat, and the two protection wing portions are located at left and right sides of a rear portion of the connecting portion, so that a first gap for accommodating the tails of the clamping pieces is formed between the protection wing portions and the connecting portion. A front end surface of the clamping piece fixing seat and the radial pin portions are matched to restrict a front and back movement range of the tails of the clamping pieces relative to the clamping piece fixing seat. The connecting portion and the protection wing portions are matched to restrict a left and right movement range of the tails of the clamping pieces relative to the clamping piece fixing seat. In this way, the movement range of the tails of the clamping pieces is restricted by arrangement of the protection wing portions, which not only facilitates the installation of the clamping pieces, but also facilitates a surgical operation.

Preferably, the front end of the clamping piece fixing seat extends forwards to form the protection wing portions, and a second gap for allowing the tails of the clamping pieces to be inserted into is formed between the protection wing portions and the radial pin portions. In this way, a simple structure is provided, and convenient installation of the clamping pieces is accomplished.

Preferably, outer side ends of the radial pin portions extend backwards to form the protection wing portions, and a third gap is formed between the protection wing portions and the front end surface of the clamping piece fixing seat. In this way, as the protection wing portions are formed on the radial pin portions, lengths of the tails of the clamping pieces may be further shortened, so that lengths of the clamping pieces and the clamp tube as well as a length of the clip placed in a patient's body may thereby be shortened.

In the first preferred solution, the two outwardly-protruding protruding portions are arranged at the tails of the clamping pieces, and when the tails of the clamping pieces are accommodated in the first gap, the two protruding portions are located at two sides of the protection wing portions, so that a rotation range of the tails of the clamping pieces relative to the clamping piece fixing seat is restricted.

In the second preferred solution, a cross-sectional shape of each of the radial pin portions is non-circular, and a shape of the deformation hole at the tails of the clamping pieces is matched with the shape of each of the radial pin portions, so that a rotation range of the tails of the clamping pieces relative to the clamping piece fixing seat is restricted. In this way, each of the radial pin portions of the clamping piece fixing seat is non-circular, such as ellipse, diamond, etc., so that the rotation of the clamping pieces is restricted. In this way, three-dimensional positioning of the clamping pieces and the clamping piece fixing seat is implemented, and that operation of a surgical procedure is facilitated through such a simple and reliable structure and high clamping precision provided by the clamping pieces. Further, when the two clamping pieces perform clamping, since the clamping pieces are connected to the stopping steps in a clamping manner through the two protruding portions, stable and reliable clamping is provided.

Preferably, a blocking piece portion restricting the clamping piece fixing seat from sliding out of the clamp tube is arranged at a front portion of the clamp tube, and the blocking piece portion is formed by bending part of a wall extending from a front end of the clamp tube. In this way, a welding process may be saved, and manufacturing costs may be lowered.

Preferably, each of the clamping pieces includes a front arm and a rear arm, an end portion of the front arm is a claw end, and shoulder portions are arranged at two wings of the front arm. The claw ends are sawtooth-shaped or non-sawtooth-shaped (e.g., a flat mouth, etc.), and the claw ends are engaged with each other when the two clamping pieces perform clamping. The front arm and the rear arm of the clamping piece are connected to each other through an arched connecting portion. An end portion of the rear arm is the tail of the clamping piece, the deformation hole of the tails of the clamping pieces is a "C"-shaped opening, and an opening direction is towards the rear end. The rear arms of the clamping pieces are accommodated in the clamp tube, and the blocking piece portion is located between the two clamping pieces. The clamping piece fixing seat is preferably made of castings, but may also be made by other mechanical processing methods. A cross-sectional shape of each of the radial pin portions may be circular, elliptical, square, rectangular, polygonal, or other special shapes, etc., but it is preferably non-circular so that the effect of rotation restricting of the clamping pieces may be implemented. Preferably, the clamp tube and the clamping pieces are made of metal blanking parts, the blocking piece portion on the clamp tube is formed by later bending, and the protruding portions on the clamping pieces may be formed together with the clamping pieces or formed by later bending.

A clamp device in cooperation with an endoscope includes an operating portion, a releasing portion, and the clamping portion as described above.

Preferably, a distal end of the mandrel in the operating portion is fixedly connected to the connecting tube in the clamping portion. A spring hose in the releasing portion is arranged on the mandrel in a sheathed manner, a proximal end of the spring hose is connected to the operating portion, and a swivel seat in the releasing portion is fixed by a distal end of the spring hose. A swivel is installed on the swivel seat, and the swivel is detachably connected to the clamp tube in the clamping portion. The operating portion pulls the mandrel to drive the clamping piece fixing seat to move backwards, so that the clamping pieces perform clamping, and the shoulder portions of the clamping pieces are restricted by the clamp tube. The mandrel is continuously pulled, the radial pin portions on the clamping piece fixing seat deform the deformation hole of the tails of the clamping pieces and separate the two, the tails of the clamping pieces are elastically expanded, and the protruding portions of the tails of the clamping pieces are buckled on the stopping steps of the clamp tube. Releasing and locking of a clip is thereby implemented.

Preferably, the operating portion includes the mandrel, a rotating wheel, a pushing tube, a sliding handle, and a handle. The sliding handle slides on the handle, the sliding handle is connected to a proximal end of the mandrel through the pushing tube, and the rotating wheel is arranged on the mandrel in a sheathed manner and drives the mandrel to rotate. The mandrel moves back and forth in a flat hole of the rotating wheel, and the proximal end of the spring hose in the releasing portion is fixedly connected to the handle. The releasing portion includes the swivel, the swivel seat, a hook, and the spring hose, and the swivel is arranged on the swivel seat through restricting steps. At least two first hook holes are arranged at the tail of the clamp tube, and at least two second hook holes are arranged at a front portion of the swivel. The hook is arranged on the mandrel in a sheathed manner and slides freely on the mandrel. The hook includes at least two hook arms, hook claws are arranged at front ends of the at least two hook arms, and the hook claws of the hook fix the clamp tube and the swivel after penetrating the first hook hole and the second hook hole corresponding to each other. The mandrel drives the connecting tube or the clamping piece fixing seat to be taken away from the hook to separate the clamp tube from the swivel. A hook middle hole is arranged at a middle portion of the hook, a hole diameter of the hook middle hole is greater than an outer diameter of the mandrel, and the hole diameter of the hook middle hole is less than an outer diameter of the connecting tube or a maximum outer diameter of the clamping piece fixing seat.

### Beneficial Effects of Disclosure

### Beneficial Effects

The foregoing technical solutions provided by the disclosure may achieve the following effects: 1. During the separation process, the tails of the clamping pieces bounce symmetrically, no parts are broken from beginning to end, and no fragments remain. 2. The tails of the clamping pieces are connected to the releasing portion to maximize the use of space, and after the clamping pieces are released, the total length is the shortest. 3. The arched connecting portion is arranged on the clamping pieces, and the clamping force thereby increases and stable clamping is provided. 4. The clamp tube covers the sharp parts of the tails of the clamping pieces, so that the parts remaining in the patient's body is smooth and have no edges and corners, and the patient's tissues are prevented from being damaged. 5. Connecting pins and stopping pins are not required, so fewer parts are need, and a stable structure is provided. 6. The symmetrically raised double protruding barbs at the tails of the clamping pieces provide higher stability.

### BRIEF DESCRIPTION OF THE DRAWINGS

### Description of Accompanying Drawings

FIG. 1 is a schematic view of a structure of a clip before being clamped in embodiment 1.
FIG. 2 is a schematic view of the structure of the clip after being clamped before being released in embodiment 1.
FIG. 3 is a schematic view of the structure of the clip after being released in embodiment 1.
FIG. 4 is a schematic view of structures of a swivel, a swivel fixing seat, a clamping piece fixing seat, a clamp tube, and a clamping piece after the clip is released in embodiment 1.
FIG. 5 is a schematic view of the structures of the clamp tube and the clamping piece after the clip is released in embodiment 1.
FIG. 6 is a schematic view of the structure of the clamp tube in embodiment 1.
FIG. 7 is a cross-sectional view of the clamp tube in embodiment 1.
FIG. 8 is a schematic view of the structure of the clamping piece in embodiment 1.
FIG. 9 is a three-dimensional view of the clamping piece fixing seat in embodiment 1.
FIG. 10 is a schematic view of the structure of the clamping piece fixing seat in embodiment 1.
FIG. 11 is a schematic view of the structure of matching between the clamping piece and the clamping piece fixing seat of the clip in embodiment 1.
FIG. 12 is a schematic view of installation of the clamping piece in embodiment 1.
FIG. 13 is a schematic view of the structure of the clamping piece fixing seat in embodiment 2.
FIG. 14 is a three-dimensional view of the clamping piece fixing seat in embodiment 2.
FIG. 15 is a schematic view of the structure of matching between the clamping piece and the clamping piece fixing seat in embodiment 2.

Herein, 1: clamping piece, 2: clamp tube, 3: clamping piece fixing seat, 4: connecting tube, 5: swivel, 6: swivel seat, 7: mandrel, 8: hook, 9: spring hose, 10: rotating wheel, 11: pushing tube, 12: sliding handle, 13: handle;
110: claw end, 120: front arm, 121: shoulder portion, 130: rear arm; 131: arched connecting portion; 140: tail; 141: deformation hole; 142: protruding portion;
210: first hook hole; 220: stopping step; 230: blocking piece portion; 510: second hook hole
300: connecting portion; 310: radial pin portion, 320: protection wing portion; 321: first gap; 322: second gap; 323: third gap.

### DESCRIPTION OF THE EMBODIMENTS

### DESCRIPTION OF THE EMBODIMENTS

The embodiments of the disclosure are provided in detail as follows. Examples of the embodiments are shown in the drawings, in which the same or similar reference numerals indicate the same or similar elements or elements with the same or similar functions throughout. The embodiments described below with reference to the accompanying drawings are exemplary, and are intended to explain the disclosure, but should not be construed as limiting the disclosure.

In the description of the disclosure, it should be understood that the terms "center", "perpendicular", "transverse", "length", "width", "thickness", "up", "down", "front", "rear", "left", "right", "vertical", "horizontal", "top", "bottom", "inner", "clockwise", "counterclockwise", and other indication orientations or positional relationships are based on the orientations and positional relationships shown in the drawings, are provided to facilitate the description of the disclosure and simplify the description, and are not intended to indicate or imply that the indicated device or element must have a specific orientation or be constructed and operated in a specific orientation, so cannot be understood as limiting the disclosure.

Further, the terms "first" and "second" are only used for descriptive purposes and cannot be understood as indicating or implying relative importance or implicitly indicating the number of indicated technical features. Thus, the features defined with "first" and "second" may explicitly or implicitly include one or more of these features. In the description of the disclosure, unless otherwise specified, "plurality" means two or more than two, unless specifically defined otherwise.

In the disclosure, unless clearly specified and defined otherwise, the terms "installed", "connected", "connecting", "fixed" and other terms should be understood in a broad sense, for instance, it can be a fixed connection, a detachable connection, or an integral connection, it can be a mechanical connection or an electrical connection, it can be a direct connection or indirect connection through an intermediate medium, and it can be the internal communication between two elements. For a person having ordinary skill in the art, the specific meaning of the above-mentioned terms in the disclosure can be understood according to specific circumstances.

In the disclosure, unless otherwise clearly specified and defined, the first feature being "above" or "below" the second feature may include direct contact between the first and second features or may include the first and second features not being in direct contact but being in contact through other features between them. Further, the first feature being "on", "above", and "upon" the second feature include that the first feature is directly above and obliquely above the second feature, or it may simply mean that the first feature is higher in level than the second feature. The first feature being "under", "below", and "beneath" the second feature include that the first feature is directly below and obliquely below the second feature, or it may simply mean that the first feature is lower in level than the second feature.

For the convenience of description, in the disclosure, the direction in which two clamping pieces in an axial direction of the clamp tube extend and expand is regarded as the front and the opposite direction is regarded as the back, and similarly, ends of other components are regarded as front ends or distal ends as close to a clamping portion, and vice versa as rear ends or proximal ends.

### Embodiment 1:

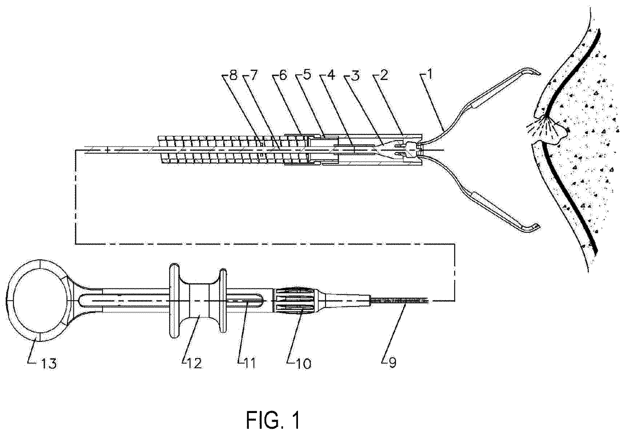

A clamp device in cooperation with an endoscope as shown in FIGs. 1 to 3 includes an operating portion, a releasing portion, and a clamping portion. Structural operations of the operating portion and the releasing portion may be referred to the patent literature with the publication number of CN108013914A. Specific description is provided as follows:

A distal end of a mandrel 7 in the operating portion is fixedly connected to a connecting tube 4 in the clamping portion. A spring hose 9 in the releasing portion is arranged on the mandrel 7 in a sheathed manner, a proximal end of the spring hose 9 is connected to the operating portion, and a swivel seat 6 in the releasing portion is fixed by a distal end of the spring hose 9. A swivel 5 is installed on the swivel seat 6, and the swivel 5 is detachably connected to a clamp tube 2 in the clamping portion. The operating portion pulls the mandrel 7 to drive the clamping piece fixing seat 3 to move backwards, so that clamping pieces 1 perform clamping, shoulder portions of the clamping pieces 1 are restricted by the clamp tube 2. The mandrel 7 is continuously pulled, radial pin portions 310 on the clamping piece fixing seat 3 deform a deformation hole 141 of tails 140 of the clamping pieces 1 and separate the two, the tails 140 of the clamping pieces 1 are elastically expanded, and protruding portions 142 of the tails 140 of the clamping pieces 1 are buckled on stopping steps 220 of the clamp tube 2. Releasing and locking of a clip is thereby implemented.

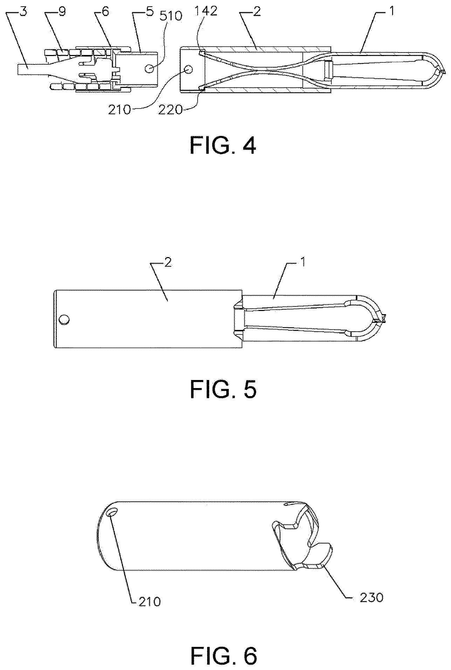

The operating portion includes the mandrel 7, a rotating wheel 10, a pushing tube 11, a sliding handle 14, and a handle 13. The sliding handle 14 slides on the handle 13, the sliding handle 14 is connected to a proximal end of the mandrel 7 through the pushing tube 11, and the rotating wheel 10 is arranged on the mandrel 7 in a sheathed manner and may drive the mandrel 7 to rotate. The mandrel 7 may move back and forth in a flat hole of the rotating wheel 10, and the proximal end of the spring hose 9 in the releasing portion is fixedly connected to the handle 13. The releasing portion includes the swivel 5, the swivel seat 6, a hook 8, and the spring hose 9, and the swivel 5 is arranged on the swivel seat 6 through restricting steps. As shown in FIG. 4 and FIG. 5, at least two first hook holes 210 are arranged at the tail of the clamp tube 2, and at least two second hook holes 510 are arranged at a front portion of the swivel 5. The hook 8 is arranged on the mandrel 7 in a sheathed manner and may slide freely on the mandrel 7. The hook 8 includes at least two hook arms, hook claws are arranged at front ends of the at least two hook arms, and the hook claws of the hook 8 fix the clamp tube 2 and the swivel 5 after penetrating the first hook hole and the second hook hole corresponding to each other. The mandrel 7 drives the connecting tube 4 or the clamping piece fixing seat 3 to be taken away from the hook 8, so as to separate the clamp tube 2 from the swivel 5. A hook middle hole is arranged at a middle portion of the hook 8, a hole diameter of the hook middle hole is greater than an outer diameter of the mandrel 7, and the hole diameter of the hook middle hole is less than an outer diameter of the connecting tube 4 or a maximum outer diameter of the clamping piece fixing seat 3. The mandrel 7 is a metal single wire or a rope and may also be a multi-stranded metal wire or a rope.

The clamping portion includes the clamping pieces 1, the clamp tube 2, the clamping piece fixing seat 3, and the connecting tube 4 configured to be connected to the mandrel 7. The clamping piece fixing seat 3 is arranged in the clamp tube 2 and can move forwards and backwards, the connecting tube 4 is fixed at a rear end of the clamping piece fixing seat 3, and the clamping pieces 1 are elastic metal pieces. The tails 140 of the two clamping pieces 1 are accommodated in the clamp tube 2, and a deformation hole 141 provided with an opening and outwardly-protruding protruding portions 142 are arranged at the tails 142 of the clamping pieces 1. The tails 140 of the clamping pieces 1 are movably connected to a front end of the clamping piece fixing seat 3 by means of the deformation hole 141, and stopping steps 220 are arranged on an inner wall of a tail of the clamp tube 2. When the two clamping pieces perform clamping, the protruding portions 142 at the tails 140 of the clamping pieces 1 are connected to the stopping steps 220 in a clamping manner. The front end of the clamping piece fixing seat 3 extends forwards to form a connecting portion 300, and a front portion of the connecting potion 300 protrudes towards left and right sides to form the radial pin portions 310. The tails of the two clamping pieces 1 are arranged on the two radial pin portions 310 in a sheathed manner via the deformation hole 141, so as to achieve movable connection between the clamping pieces 1 and the clamping piece fixing seat 3.

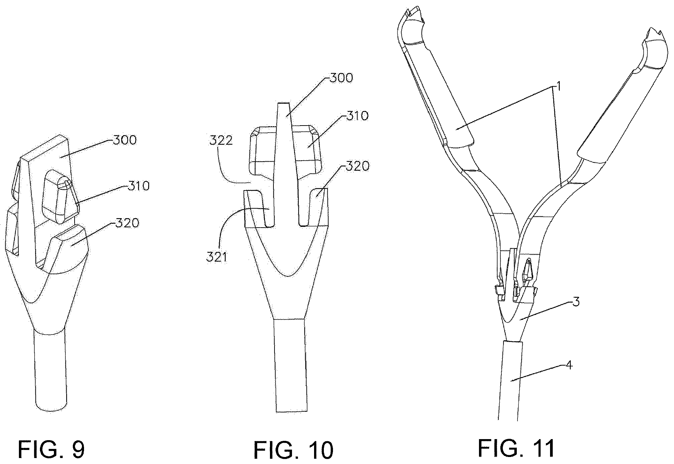

As shown in FIG. 9, FIG. 10, and FIG. 11, two protection wing portions 320 are arranged on the clamping piece fixing seat 3, and the two protection wing portions 320 are located at left and right sides of a rear portion of the connecting portion 300, so that a first gap 321 for accommodating the tails of the clamping pieces 1 is formed between the protection wing portions 320 and the connecting portion 300. A front end surface of the clamping piece fixing seat 3 and the radial pin portions 310 are matched to restrict a front and back movement range of the tails of the clamping pieces 1 relative to the clamping piece fixing seat (3). The connecting portion 300 and the protection wing portions 320 are matched to restrict a left and right movement range of the tails of the clamping pieces 1 relative to the clamping piece fixing seat 3.

As shown in FIG. 11, the two outwardly-protruding protruding portions 142 are arranged at the tails of the clamping pieces 1, and when the tails of the clamping pieces 1 are accommodated in the first gap 321, the two protruding portions 142 are located at two sides of the protection wing portions 320 so that a rotation range of the tails of the clamping pieces 1 relative to the clamping piece fixing seat 3 is restricted. In this way, three-dimensional positioning of the clamping pieces and the clamping piece fixing seat is implemented, and that operation of a surgical procedure is facilitated through such a simple and reliable structure. Further, when the two clamping pieces perform clamping, since the clamping pieces are connected to the stopping steps 220 in a clamping manner through the two protruding portions, stable and reliable clamping is provided.

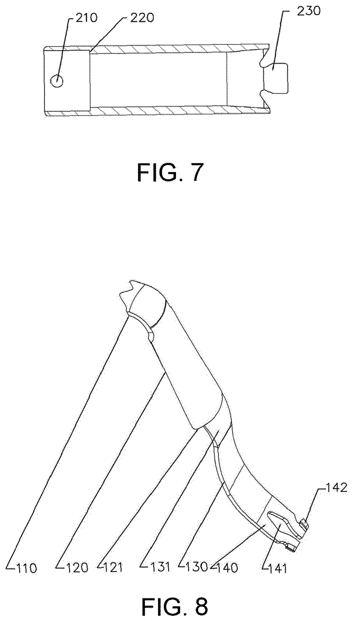

As shown in FIG. 6 and FIG. 7, a blocking piece portion 230 restricting the clamping piece fixing seat 3 from sliding out of the clamp tube 2 is arranged at a front portion of the clamp tube 2, and the blocking piece portion 230 is formed by bending part of a wall extending from a front end of the clamp tube 2. In this way, a welding process may be saved, and manufacturing costs may be lowered.

As shown in FIG. 8, each of the clamping pieces 1 includes a front arm 120 and a rear arm (130), an end portion of the front arm 120 is a claw end 110, and shoulder portions 121 are arranged at two wings of the front arm 120. The claw ends 110 are sawtooth-shaped, and the claw ends 110 are engaged with each other when the two clamping pieces 1 perform clamping. The front arm 120 and the rear arm 130 of the clamping piece 1 are connected to each other through an arched connecting portion 131. An end portion of the rear arm 130 is the tail 140 of the clamping piece 1. The deformation hole 141 of the tails 140 of the clamping pieces 1 is a "C"-shaped opening, and an opening direction is towards the rear end. The rear arms 130 of the clamping pieces 1 may be accommodated in the clamp tube 2, and the blocking piece portion 230 is located between the two clamping pieces 1. The clamping piece fixing seat is preferably made of castings, but may also be made by other mechanical processing methods. A cross-sectional shape of each of the radial pin portions 310 may be circular, elliptical, square, rectangular, polygonal, or other special shapes, etc., but it is preferably non-circular so that the effect of rotation restricting of the clamping pieces may be implemented. In other embodiments, an outer diameter (matched with an opening distance of the deformation hole at the tails of the clamping pieces) of each of the radial pin portions in a front-and-rear direction is significantly less than the outer diameter in other directions. In this way, when the clamping pieces are installed, the opening of the deformation hole at the tails of the clamping pieces may be sleeved on the radial pin portions 310 in a direction perpendicular to the front-and-rear and may then be rotated by 90°. As such, the claw ends of the clamping pieces face forwards, and the deformation hole at the tails of the clamping pieces is buckled on and connected to the radial pin portions, so installing and manufacturing may thereby be conveniently performed. The clamp tube and the clamping pieces are made of metal blanking parts, the blocking piece portion on the clamp tube is formed by later bending, and the protruding portions on the clamping pieces may be formed together with the clamping pieces or formed by later bending.

In this embodiment, the front end of the clamping piece fixing seat 3 extends forwards to form the protection wing portions 320, and a second gap 322 for allowing the tails of the clamping pieces 1 to be inserted into is formed between the protection wing portions 320 and the radial pin portions 310.

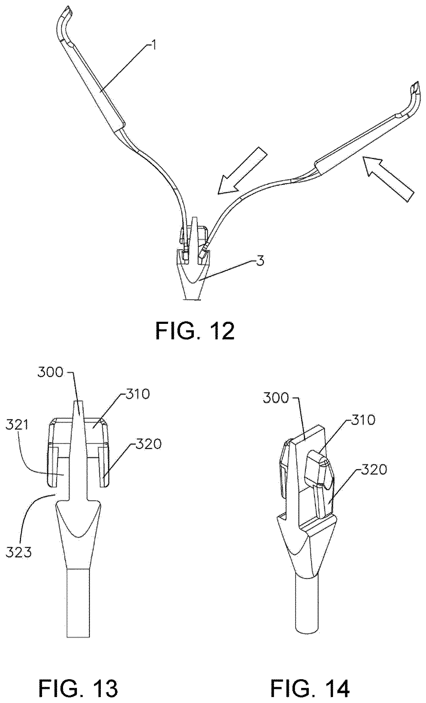

As shown in FIG. 12, when the clamping pieces 1 and the clamping piece fixing seat 2 are installed and connected, the clamping pieces 1 and the clamping piece fixing seat 3 are inserted into the second gap at a specific angle, so that connecting holes of the tails of the clamping pieces enter the radial pin portions of the clamping piece fixing seat, and the tails of the clamping pieces enter into the first gap (double U-shaped groove) of the clamping piece fixing seat. The two clamping pieces are symmetrical on both sides and are constrained radially in the tube after being retracted into the clamp tube.

When the clamp tube is installed, after the clamping pieces are housed into the clamp tube by the clamping piece fixing seat and before the two clamping pieces are closed, blocking pieces at two sides are bent inwards to form the blocking piece portions configured for blocking, so as to prevent the clamping piece fixing seat from extending out of the front end of the clamp tube again.

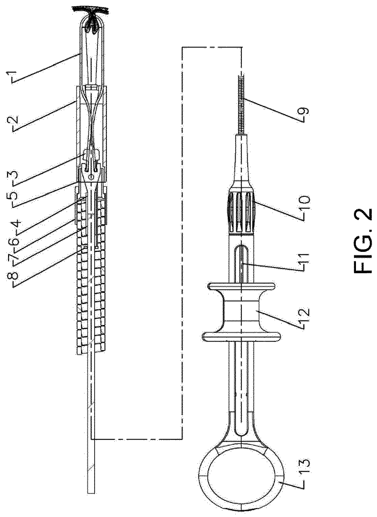

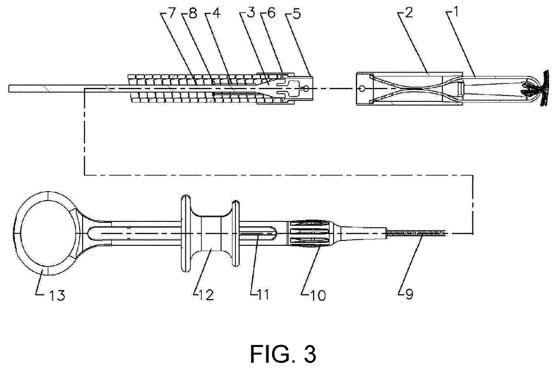

When in use, the sliding handle 12 slides in a sliding groove of the handle 13, and its forward movement may push the mandrel 7 to drive the clamping piece fixing seat 3 to move forwards, so that the clamping pieces 1 extend out of the clamp tube 2, and the clamping pieces 1 thereby expand. As shown in FIG. 1: its backward movement may pull the mandrel 7 to drive the clamping piece fixing seat 3 to move backwards, so that the clamping pieces 1 are retracted into the clamp tube 2, and the clamping pieces 1 thereby close and perform clamping. As shown in FIG. 2: the sliding handle 12 continues to move backwards, pulling the mandrel 7 to drive the clamping piece fixing seat 3 to move backwards, and the radial pin portions 310 on the clamping piece fixing seat 3 deform the deformation hole 141 and separate the tails of the clamping pieces. The tails 140 of the two clamping pieces 1 are elastically separated after being separated from the clamping piece 3, and the protruding portions 142 of the tails of the clamping pieces are buckled on the stopping steps 220 of the clamp tube 2. The mandrel 7 continues to move backwards, the clamping piece fixing seat 3 is brought away from the elastic hook 8 to separate the clamp tube 2 from the swivel 5. As shown in FIG. 3, the clip is released, no parts are broken from beginning to end, and no fragments remain.

### Embodiment 2:

A clamp device in cooperation with an endoscope includes an operating portion, a releasing portion, and a clamping portion. The only difference between this embodiment and embodiment 1 is the following structure of the clamping portion:

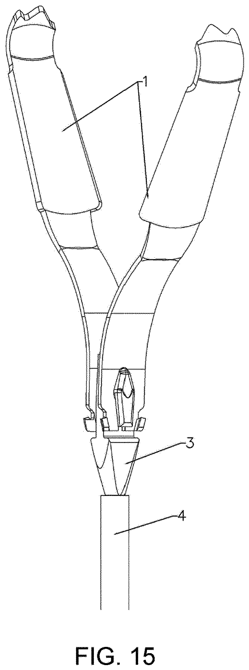

As shown in FIG. 13, FIG. 14, and FIG. 15, outer side ends of the radial pin portions 310 extend backwards to form the protection wing portions 320, and a third gap 323 is formed between the protection wing portions 320 and the front end surface of the clamping piece fixing seat 3. In this way, as the protection wing portions are formed on the radial pin portions, lengths of the tails of the clamping pieces may be further shortened, so that lengths of the clamping pieces and the clamp tube as well as a length of the clip placed in a patient's body may thereby be shortened. Others are the same as in embodiment 1.

In the description of the specification, descriptions with reference to the terms such as "one embodiment", "some embodiments", "examples", "specific examples", or "some examples" etc. means that the specific features, structures, materials, or characteristics described in combination with the embodiment(s) or example(s) are included in at least one embodiment or example of the disclosure. In the specification, the schematic representation of the above-mentioned terms does not necessarily refer to the same embodiment or example. Further, the described specific features, structures, materials, or characteristics may be combined in any one or more embodiments or examples in a suitable manner.

Although the embodiments of the disclosure have been shown and described above, it can be understood that the above-mentioned embodiments are exemplary and should not be construed as limiting the disclosure. A person having ordinary skill in the art may make changes, corrections, substitutions, and modifications to the above-mentioned embodiments within the scope of the disclosure without departing from the principle and purpose of the disclosure. Any modifications, equivalent replacements, and improvements made without departing from the spirit and principles of the disclosure should fall within the protection scope of the disclosure.

## Claims

1. A clamping portion of a clamp device in cooperation with an endoscope, comprising clamping pieces (1), a clamp tube (2), a clamping piece fixing seat (3), and a connecting tube (4) configured to be connected to a mandrel (7), wherein the clamping piece fixing seat (3) is arranged in the clamp tube (2) and moves forwards and backwards, the connecting tube (4) is fixed at a rear end of the clamping piece fixing seat (3), the clamping pieces (1) are elastic metal pieces, tails (140) of the two clamping pieces (1) are accommodated in the clamp tube (2), a deformation hole (141) provided with an opening and outwardly-protruding protruding portions (142) are arranged at the tails (142) of the clamping pieces (1), the tails (140) of the clamping pieces (1) are movably connected to a front end of the clamping piece fixing seat (3) by means of the deformation hole (141), stopping steps (220) are arranged on an inner wall of a tail of the clamp tube (2), and when the two clamping pieces perform clamping, the protruding portions (142) at the tails (140) of the clamping pieces (1) are connected to the stopping steps (220) in a clamping manner, wherein a connecting portion (300) is arranged at the front end of the clamping piece fixing seat (3), a front portion of the connecting portion (300) protrudes towards left and right sides to form radial pin portions (310), the tails of the two clamping pieces (1) are arranged on the two radial pin portions (310) in a sheathed manner via the deformation hole (141), so as to achieve movable connection between the clamping pieces (1) and the clamping piece fixing seat (3).

2. The clamping portion of the clamp device in cooperation with the endoscope according to claim 1, wherein two protection wing portions (320) are arranged on the clamping piece fixing seat (3), the two protection wing portions (320) are located at left and right sides of a rear portion of the connecting portion (300), so that a first gap (321) for accommodating the tails of the clamping pieces (1) is formed between the protection wing portions (320) and the connecting portion (300), a front end surface of the clamping piece fixing seat (3) and the radial pin portions (310) are matched to restrict a front and back movement range of the tails of the clamping pieces (1) relative to the clamping piece fixing seat (3), and the connecting portion (300) and the protection wing portions (320) are matched to restrict a left and right movement range of the tails of the clamping pieces (1) relative to the clamping piece fixing seat (3).

3. The clamping portion of the clamp device in cooperation with the endoscope according to claim 2, wherein the front end of the clamping piece fixing seat (3) extends forwards to form the protection wing portions (320), and a second gap (322) for allowing the tails of the clamping pieces (1) to be inserted into is formed between the protection wing portions (320) and the radial pin portions (310).

4. The clamping portion of the clamp device in cooperation with the endoscope according to claim 2, wherein outer side ends of the radial pin portions (310) extend backwards to form the protection wing portions (320), and a third gap (323) is formed between the protection wing portions (320) and the front end surface of the clamping piece fixing seat (3).

5. The clamping portion of the clamp device in cooperation with the endoscope according to claim 2, wherein the two outwardly-protruding protruding portions (142) are arranged at the tails of the clamping pieces (1), and when the tails of the clamping pieces (1) are accommodated in the first gap (321), the two protruding portions (142) are located at two sides of the protection wing portions (320) so that a rotation range of the tails of the clamping pieces (1) relative to the clamping piece fixing seat (3) is restricted.

6. The clamping portion of the clamp device in cooperation with the endoscope according to claim 1, wherein a cross-sectional shape of each of the radial pin portions (310) is non-circular, and a shape of the deformation hole (141) at the tails of the clamping pieces (1) is matched with the shape of each of the radial pin portions (310), so that a rotation range of the tails of the clamping pieces (1) relative to the clamping piece fixing seat (3) is restricted.

7. The clamping portion of the clamp device in cooperation with the endoscope according to claim 1, wherein a blocking piece portion (230) restricting the clamping piece fixing seat (3) from sliding out of the clamp tube (2) is arranged at a front portion of the clamp tube (2), and the blocking piece portion (230) is formed by bending part of a wall extending from a front end of the clamp tube (2).

8. The clamping portion of the clamp device in cooperation with the endoscope according to claim 7, wherein: each of the clamping pieces (1) comprises a front arm (120) and a rear arm (130), an end portion of the front arm (120) is a claw end (110), shoulder portions (121) are arranged at two wings of the front arm (120), the claw ends (110) are engaged with each other when the two clamping pieces (1) perform clamping, the front arm (120) and the rear arm (130) of the clamping piece (1) are connected to each other through an arched connecting portion (131), an end portion of the rear arm (130) is the tail (140) of the clamping piece (1), the deformation hole (141) of the tails (140) of the clamping pieces (1) is a "C"-shaped opening, an opening direction is towards the rear end, the rear arms (130) of the clamping pieces (1) are accommodated in the clamp tube (2), and the blocking piece portion (230) is located between the two clamping pieces (1).

9. A clamp device in cooperation with an endoscope, comprising an operating portion, a releasing portion, and the clamping portion according to any one of claims 1 to 8.

10. The clamp device in cooperation with the endoscope according to claim 9, wherein: a distal end of the mandrel (7) in the operating portion is fixedly connected to the connecting tube (4) in the clamping portion, a spring hose (9) in the releasing portion is arranged on the mandrel (7) in a sheathed manner, a proximal end of the spring hose (9) is connected to the operating portion, a swivel seat (6) in the releasing portion is fixed by a distal end of the spring hose (9), a swivel (5) is installed on the swivel seat (6), and the swivel (5) is detachably connected to the clamp tube (2) in the clamping portion; wherein the operating portion pulls the mandrel (7) to drive the clamping piece fixing seat (3) to move backwards so that the clamping pieces (1) perform clamping, the clamping pieces (1) are restricted by the clamp tube (2), the mandrel (7) is continuously pulled, the radial pin portions (310) on the clamping piece fixing seat (3) deform the deformation hole (141) of the tails (140) of the clamping pieces (1) and separate the two, the tails (140) of the clamping pieces (1) are elastically expanded, and the protruding portions (142) of the tails (140) of the clamping pieces (1) are buckled on the stopping steps (220) of the clamp tube (2); the operating portion comprises the mandrel (7), a rotating wheel (10), a pushing tube (11), a sliding handle (14), and a handle (13), the sliding handle (14) slides on the handle (13), the sliding handle (14) is connected to a proximal end of the mandrel (7) through the pushing tube (11), the rotating wheel (10) is arranged on the mandrel (7) in a sheathed manner and drives the mandrel (7) to rotate, the mandrel (7) moves back and forth in a flat hole of the rotating wheel (10), the proximal end of the spring hose (9) in the releasing portion is fixedly connected to the handle (13), the releasing portion comprises the swivel (5), the swivel seat (6), a hook (8), and the spring hose (9), the swivel (5) is arranged on the swivel seat (6) through restricting steps, at least two first hook holes are arranged at the tail of the clamp tube (2), at least two second hook holes are arranged at a front portion of the swivel (5), the hook (8) is arranged on the mandrel (7) in a sheathed manner and slides freely on the mandrel (7), the hook (8) comprises at least two hook arms, hook claws are arranged at front ends of the at least two hook arms, the hook claws of the hook (8) fix the clamp tube (2) and the swivel (5) after penetrating the first hook holes and the second hook holes corresponding to each other, and the mandrel (7) drives the connecting tube (4) or the clamping piece fixing seat (3) to be taken away from the hook (8) to separate the clamp tube (2) from the swivel (5); wherein a hook middle hole is arranged at a middle portion of the hook (8), a hole diameter of the hook middle hole is greater than an outer diameter of the mandrel (7), and the hole diameter of the hook middle hole is less than an outer diameter of the connecting tube (4) or a maximum outer diameter of the clamping piece fixing seat (3).
